Europäisches Patentamt

European Patent Office

Office européen des brevets

(11) Publication number: **0 098 632**
**B1**

(12) **EUROPEAN PATENT SPECIFICATION**

(45) Date of publication of patent specification: 03.09.86

(51) Int. Cl.⁴: **C 07 C 141/08**, C 07 C 91/10

(21) Application number: 83200853.6

(22) Date of filing: 10.06.83

(54) Salts of acid ether sulphates and process for the preparation of these salts.

(30) Priority: 12.06.82 NL 8202398

(43) Date of publication of application:
18.01.84 Bulletin 84/03

(45) Publication of the grant of the patent:
03.09.86 Bulletin 86/36

(84) Designated Contracting States:
AT BE CH DE FR GB IT LI LU NL SE

(56) References cited:
GB-A- 891 631

CHEMICAL ABSTRACTS, vol. 96, no. 2, 11th
January 1982, page 114, no. 8526d, Columbus,
Ohio, USA

(73) Proprietor: STAMICARBON B.V.
Mijnweg 1
NL-6167 AC Geleen (NL)

(72) Inventor: Van Paassen, Nicolaas Adrianus
Ignatius
Beiershof 13
NL-2411 JR Bodegraven (NL)
Inventor: Verschuur, Jacobus Gerardus
H. Goebelstraat 19
NL-2411 AT Bodegraven (NL)

(74) Representative: Hoogstraten, Willem Cornelis
Roeland et al
OCTROOIBUREAU DSM Postbus 9
NL-6160 MA Geleen (NL)

Courier Press, Leamington Spa, England.

## Description

The invention relates to salts of acid ether sulphates that are liquid under normal temperature and pressure conditions as well as to the preparation of these salts.

It is known that such salts, which have a very good surface activity, can be prepared by neutralization of acid sulphates of the general formula $R(OCH_2CH_2)_nOSO_2OH$, where n represents a number having an average value of 2—3 and R an alkyl group with 8—16 carbon atoms with monobutylethanolamine, isopropylamine or di-ethyl amine (see British patent specification 891.631). Compared with the corresponding, also known, sodium salts, these known salts have the advantage that they do not have a pasty consistency under normal temperature and pressure conditions but are liquid, which not only cuts down transport cost but also is important for water-free applications of said salts. A disadvantage of these known salts is, however, that their odour and colour are not ideal, rendering these salts less suitable for various applications. In addition it is difficult to dilute them with water because gel formation takes place.

It has now been found that neutralization of various acid ether sulphates with tri-isopropanolamine may yield salts that not only have a suitable odour and colour but also are liquid under normal temperature and pressure conditions and furthermore can properly be dissolved in water without gel formation occurring.

The salts according to the invention are salts of acid sulphates of the general formula $R(OCH_2CH_2)_nOSO_2OH$ where n represents a number having an average value of 1—6 and R an oleyl group or an alkyl group with 8—16 carbon atoms with tri-isopropanolamine.

Particularly suitable are the salts according to the invention where n represents a number having an average value of 1.5—3.5, for instance the salt in which R represents a lauryl group and n an average value of 2.

The acid sulphates of the said general formula can be obtained in a known way by condensing the corresponding alcohol ROH with epoxyethane in an amount corresponding with the desired average number of ethoxy groups and sulphating the reaction product formed. If a mixture of alcohols, for instance technical grade lauryl alcohol, is started from, the corresponding mixture of acid sulphates is obtained that can be converted to the corresponding mixture of salts according to the invention using tri-isopropanolamine. From the reaction product obtained by sulphation of the product formed upon condensation of the alcohol or the mixture of alcohols with epoxyethane, the di- and/or triethoxy compound, for instance, may be isolated so that it can be applied for neutralization with tri-isopropanolamine.

The neutralization of the acid sulphate with triisopropanolamine may for instance be effected by gradually adding the melted amine (melting point 58°C) to the acid sulphate whilst stirring. The neutralization can be effected using reflux cooling or under pressure in a closed system. The temperature then is preferably chosen to be 35—45°C, because this makes stirring of the reaction mixture easier than at normal ambient temperatures. For neutralization of a certain amount of acid sulphate, such an amount of tri-isopropanolamine is needed as to give an aqueous solution of the end product obtained a pH of about 5.5. As the tri-isopropanolamine is not a strong base and the acid sulphate has a strongly acid character, and because of the presence of acid impurities, this neutralization requires an amount of amine that is larger than the stoichiometric amount.

The salts according to the invention can be applied to various purposes, for instance as hair washing agent or as emulsifier in anhydrous systems such as anhydrous insecticide preparations.

The invention will be elucidated in the following examples. The salts obtained according to these examples are practically odourless and have a light-yellow to yellow colour.

### Example I

Technical-grade octyl alcohol is condensed with 3.3 moles epoxyethane per mole alcohol. While being stirred, the product obtained (345 g) is sulphated with 150 g $HSO_3Cl$, which is gradually added in 30 minutes' time. The temperature increases to about 33°C. The reaction mixture obtained is subsequently kept at 40°C for 40 minutes while being stirred. An amount of 451 g acid sulphate is obtained, the Epton value of which is calculated to be 2.77 milligramme equivalents per g.

Of the acid sulphate obtained, 448.5 g is neutralized with 267 g tri-isopropanolamine (hereinafter referred to as tipa) at 40°C. Thus, 713 g salt with an Epton value of 1.58 mgeq/g is obtained. After addition of 4 g acetic acid and dilution with water of the product obtained to a concentration of 10 wt.-%, the pH of the aqueous solution is 5.1.

### Example II

In 30 minutes 120 g $HSO_3Cl$ is gradually added to 348 g of a condensation product that is commercially available under the name Synperonic (3 moles epoxyethane per mole of a mixture of $C_{11}$—$C_{13}$ alcohols, available under the name synprol), which is meanwhile being stirred. The temperature increases to 35°C.

While being stirred, and while nitrogen is passed through it, the resulting mixture is subsequently kept at 38°C for 40 minutes. An amount of 433.5 g acid sulphate is obtained, with a calculated Epton value of 2.32 mgeq/g.

At a temperature of 40°C, 404.5 g of the acid sulphate is neutralized with 211.5 g tipa. An amount of 616 g salt is obtained, the Epton value of which is calculated to be 1.43 mgeq/g. After dilution with water to a concentration of 10 wt.-%, the salt has a pH of 5.2.

## Example III

In 3.5 hours' time 1489 g HSO₃Cl is gradually added to 2565 g of the product of condensation of technical-grade lauryl alcohol with 2 moles epoxyethane per mole alcohol, which is meanwhile being stirred. The temperature increases to about 35°C. While being stirred, and while nitrogen is passed through it, the mixture obtained is subsequently kept at 40°C for 45 minutes. An amount of 3329 g acid sulphate is obtained, the Epton value of which is calculated to be 2.7 mgeq/g.

Of the acid sulphate obtained, 3275 g is neutralized with 1885 g tipa at about 40°C. The yield is 5160 g, with an Epton value of 1.61 mgeq/g. The pH of the water-diluted salt (10 wt.-%) is 5.55.

## Example IV

In 35 minutes' time 118 g HSO₃Cl is gradually added to 392 g of the product of condensation of technical-grade oleyl alcohol with 3.2 moles epoxyethane per mole alcohol, which is meanwhile being stirred. The temperature increases to about 35°C. While being stirred, and while nitrogen is passed through it, the mixture obtained is subsequently kept at 40°C for 30 minutes. An amount of 486 g acid sulphate is obtained, the Epton value of which is calculated to be 2.02 mgeq/g.

Of the acid sulphate obtained, 406 g is neutralized with 207 g tipa at 35—40°C. An amount of 613 g salt is obtained, the Epton value being 1.25 mgeq/g. The pH of the water-diluted salt (10 wt.-%) is 5.2.

## Claims

1. Salts of acid sulphates of the general formula $R(OCH_2CH_2)_nOSO_2OH$, where n represents a number having an average value of 1—6 and R an oleyl group or an alkyl group with 8—16 carbon atoms, with tri-isopropanolamine.

2. Salts according to claim 1, wherein n represents a number with an average value of 1.5—3.5.

3. Salts according to claim 2, wherein R represents a lauryl group and n an average value of 2.

4. Process for the preparation of salts according to any one of claims 1—3, characterized in that the acid sulphate in question is neutralized with tri-isopropanolamine.

## Patentansprüche

1. Salze von saueren Sulfaten der allgemeinen Formel $R(OCH_2CH_2)_nOSO_2OH$, worin n eine Zahl mit einem Durchschnittswert von 1—6 und R eine Oleylgruppe oder eine Alkylgruppe mit 8—16 Kohlenstoffatomen darstellt, mit Triisopropanolamin.

2. Salze nach Anspruch 1, in denen n eine Zahl mit einem Durchschnittswert von 1,5—3,5 darstellt.

3. Salze nach Anspruch 2, worin R eine Laurylgruppe und n einen Durchschnittswert von 2 darstellt.

4. Verfahren zur Herstellung von Salzen nach einem der Ansprüche 1—3, dadurch gekennzeichnet, daß das fragliche sauere Sulfat mit Triisopropanolamin neutralisiert wird.

## Revendications

1. Sels de sulfates acides de formule générale $R(OCH_2CH_2)_nOSO_2OH$, dans laquelle n est un nombre ayant un valeur moyenne de 1 à 6 et R est un radical oléyle ou un radical alkyle de 8 à 16 atomes de carbone, avec la tri-isopropanolamine.

2. Sels selon la revendication 1, dans lesquels n est un nombre ayant une valeur moyenne de 1,5 à 3,5.

3. Sels selon la revendication 2, dans lesquels R représente un radical lauryle et n a une valeur moyenne de 2.

4. Procédé de préparation de sels selon l'une quelconque des revendications 1 à 3 caractérisé en ce qu'on neutralise le sulfate acide en question avec la tri-isopropanolamine.